(19) Europäisches Patentamt
European Patent Office
Office européen des brevets

(11) EP 1 935 393 B1

(12) **EUROPÄISCHE PATENTSCHRIFT**

(45) Veröffentlichungstag und Bekanntmachung des Hinweises auf die Patenterteilung:
**25.02.2015 Patentblatt 2015/09**

(51) Int Cl.:
***A61K 6/09*** *(2006.01)* ***A61K 6/083*** *(2006.01)*

(21) Anmeldenummer: **07023518.9**

(22) Anmeldetag: **05.12.2007**

(54) **Dentalkomposite mit Tricyclo[5.2.1.02.6]decan-Derivaten**

Dental composites with Tricyclo[5.2.1.02.6]decane derivatives

Composite dentaire doté de dérivés de tricyclo[5.2.1.02.6]decane

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **20.12.2006 DE 102006060983**

(43) Veröffentlichungstag der Anmeldung:
**25.06.2008 Patentblatt 2008/26**

(73) Patentinhaber: **Heraeus Kulzer GmbH**
**63450 Hanau (DE)**

(72) Erfinder:
- **Utterodt, Andreas, Dr.**
  **61267 Neu Anspach (DE)**
- **Ruppert, Klaus, Dr.**
  **63477 Maintal (DE)**
- **Schaub, Matthias, Dr.**
  **63589 Linsengericht (DE)**
- **Diefenbach, Christine**
  **65599 Dornburg (DE)**
- **Reischl, Kurt**
  **35799 Merenberg (DE)**
- **Hohmann, Alfred**
  **61389 Schmitten (DE)**
- **Eck, Michael**
  **61389 Schmitten (DE)**
- **Schönhof, Nelli**
  **35619 Braunfels (DE)**

(74) Vertreter: **Bendele, Tanja et al**
**RUHR-IP**
**Postfach 23 01 44**
**45069 Essen (DE)**

(56) Entgegenhaltungen:
**EP-A- 0 254 185** **EP-A- 0 497 157**
**EP-A1- 1 719 497**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 935 393 B1

## Beschreibung

Die Erfindung betrifft die Verwendung Urethangruppen enthaltender Acrylsäureester des Tricyclo[5.2.1.02.6] decans.

### *Stand der Technik*

Als schrumpfarm polymerisierende Monomere für Zahnfüllungsmaterialien haben sich Bisphenol A-(meth)acrylatmonomere bewährt. Eine Alternative zu den schrumpfarm polymerisierenden Bisphenol A-(meth)acrylatmonomeren wurde mit TCD-Monomeren in der EP 0 254 185 (Bayer AG) beschrieben. Wie das Bisphenol-A- Gerüst weist die TCD-Gruppe die Steifheit auf, die dann das schrumpfarme Polymerisationsverhalten bedingt. Durch die sterische Einschränkung der Beweglichkeit sind auch die Urethanderivate vom 1,3-Bis(1-isocyanato-1-methylethyl)-benzol in ihren Eigenschaften dem Bis-GMA sehr ähnlich und können an seiner Stelle in Dentalkompositen eingesetzt werden, wie die EP 0 934 926 beschreibt.

Konkret wird dann allerdings nur die Verwendung der Methacrylate beschrieben.

Die sogenannten Silorane stellen eine Kombination von Epoxidfunktionalitäten an Siloxaneinheiten dar und können über einen kationischen Vernetzungsmechanismus durch Ringöffnungspolymerisation schrumpfarm polymerisiert werden. Der geringe Schrumpf dieser neuen Monomere und die toxikologische Unbedenklichkeit der sonst kritischen Epoxide in ausgehärteten Dentalkompositen wurde beschrieben in DE 100 01 228 und EP 1 117 368.

EP1719497A1 betriff die Herstellung von Kompositmaterialien mit geringer Schrumpfkraft mit 0,5-10 Gew.-% Nanofüller, größer gleich 60 Gew.-% Füllstoffgemisch und 1 Gew.-% Initiator und einem verbleibenden Anteil an Monomerkomponente, die zu 60 bis 80 Gew.-% Bis-GMA oder TCD-di-HEMA oder TCD-di-HEA umfasst.

### *Ausgangslage*

Die im Vergleich mit Methacrylaten höhere Reaktivität von Acrylatmonomeren ist bekannt, jedoch ist auch die Reizwirkung gegenüber biologischem Gewebe deutlich höher als die von Methacrylaten, weshalb in Dentalmaterialien vorwiegend Monomermischungen mit Methacrylaten, gegebenenfalls mit geringen Beimengungen von Acrylaten eingesetzt werden. Die gesteigerte Reaktivität von Urethan(meth)acrylat-Monomeren gegenüber Polyether-, Polyester- oder aliphatischen Monomeren ist ebenfalls bekannt. Es stellt sich demgegenüber die Aufgabe, trotz Einsatz von Acrylatmonomeren Dentalkomposite mit günstigen Eigenschaften zur Verfügung zu stellen.

### *Detaillierte Beschreibung der Erfindung*

Die Erfindung betrifft die Verwendung eines Vernetzeranteil, der zu mehr als 50% gebildet wird von Acrylatmonomeren mit einer TCD-Urethan-Struktur die ausgewählt sind aus Acrylatmonomeren mit einer diacrylatfunktionellen TCD-Urethan-Struktur ausgewählt aus TCD-di-HEA, einem Acrylsäureester des Tricyclo[5.2.1.02.6]decans zur Herstellung einer Dentalkomposit-Zusammensetzung enthaltend Monomere, Vernetzer, Füllstoffe, Initiatoren , wobei die Cytotoxizität des gehärteten Komposits entsprechend den Normvorgaben nach ISO 10993-5 und DIN EN ISO 7405 die Bewertung "kein zytotoxisches Potential" aufweist.

Der Vernetzeranteil wird zu mehr als 50% gebildet von Acrylatmonomeren mit einer TCD-Urethan-Struktur der allgemeinen Formel

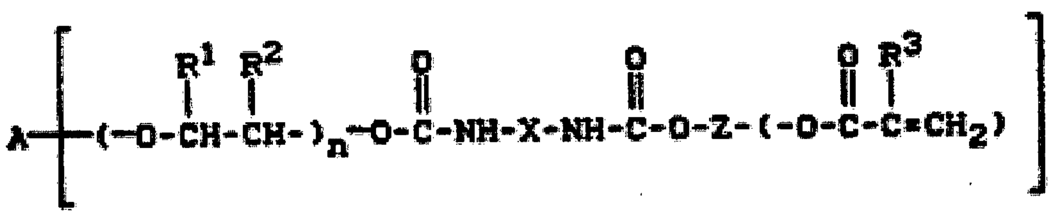
(I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
$R^1$ und $R^2$ gleich sind und Wasserstoff oder verschieden sind und Wasserstoff und

Methyl bedeuten,
n für jede von A ausgehende Kette unabhängig eine Zahl von 0 bis 5 bedeutet,
X für die Gruppe

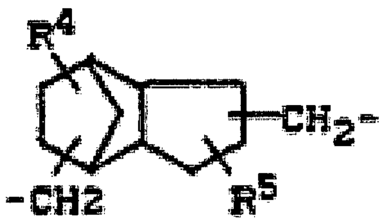

steht, in der
$R^4$ und $R^5$ gleich oder verschieden sind und Wasserstoff, Halogen, Niederalkoxy, Niederalkyl oder Trifluormethyl bedeuten,
Z einen zweiwertigen geradkettigen oder verzweigten aliphatischen Kohlenwasserstoffrest von 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, $R^2$, $R^3$ auschließlich Wasserstoff sind
und dass die Zytotoxizität entsprechend der Normvorgaben nach ISO 10993-5 und DIN EN ISO 7405 die Bewertung "kein zytotoxisches Potential" aufweist.

***Vorteile der erfindungsgemäßen Zusammensetzungen***

**A** Die hohe Reaktivität eines Urethangruppen enthaltenden Acrylsäureesters wurde mit der steifen Struktur des TCD-Gerüstes kombiniert und kann damit als Alternative zu Bis-GMA in Dentalkompositen eingesetzt werden. Dabei werden bei einer gesteigerten Reaktivität höhere Umsatzgrade erreicht und entgegen des bekannten Zusammenhanges zwischen Umsatzgrad und Volumenschwund trotzdem schrumpfarme Komposite ermöglicht. Unerwartet günstig fällt dabei die toxikologische Prüfung mit einem Acrylatharzanteil von > 5% aus (Beispiele/Anlagen).
**B** Die toxikologischen Prüfungen belegen die überraschend hohe biologische Verträglichkeit des polymerisierten Komposites.
**C** Höhere Polymerisationsgrade sind vorteilhaft für die mechanischen Eigenschaften der Komposite, allerdings galten Acrylatmonomere als ungeeignete Vernetzer aufgrund der nachteiligen toxikologischen Eigenschaften. Nach der Aushärtung wurde überraschend eine sehr günstige Biokompatibilität nachgewiesen. Die dentalen Komposite werden in der direkten und indirekten Zahnheilkunde eingesetzt.

***Beispiel***

Kompositpaste (erfindungsgemäß)

Die Formulierung erfolgt im Kneter mit Planetengetriebe. Die Arbeiten sind unter Gelblicht durchzuführen. Monomere, Initiatoren und Hilfsstoffe werden vorgelegt (evtl. bereits vorgelöst) und bei 2500 RPM 10 min homogenisiert. Der Füllstoff wird abgewogen und in mehreren Portionen mit abnehmender Menge zugegeben ([%]: 35 / 25 / 20 / 10 / 5 / 5). Nach jeder Zugabe wird erneut homogenisiert bis eine knetbare Paste entstanden ist. Bei starker Erwärmung soll die Paste vor dem nächsten Mischvorgang etwas abkühlen. Bei noch vorhandenen Füllstoffresten wird der Mischvorgang noch einmal wiederholt.

Toxilogische Prüfung (Zytotoxizitätsprüfung in vitro durch Bildung eines XXT Farbstoffes)

Mit dem XTT-Farbtest werden die Teilungsfähigkeit und die Überlebensrate der Zellen gleichzeitig über eine colorimetrische Bestimmung bewertet. Die Prüfung basiert auf der Freisetzung des gelben Tetrazoliumsalzes XTT (Natrium-3'-(1-phenylaminocarbonyl)-3,4-tetrazolium)-bis(4-methoxy-6-nitro)benzensulfonsäure-Hydrat), das einen orangefarbenen, wasserlöslichen Formazanfarbstoff bildet infolge der Dehydrogenaseaktivität von aktiven Mitochondrien.
Die Prüfung der Cytotoxizität erfolgte entsprechend der Normvorgaben nach ISO 10993-5 und DIN EN ISO 7405. Dazu wurde die unsterile Materialprobe unter Rühren für 72±2 Stunden bei 37±1 °C extrahiert (Extraktionsmittel: Dubecco's Modified Eagle Medium (DMEM) versetzt mit 10 % Fetal Calf Serum (FCS)). Das Verhältnis Oberfläche/Vo-

lumen war 6 $cm^2$/ml. Anschließend wurde der Extrakt steril filtriert.

Eine positive und eine negative Kontrolle zur Zellkultur durchlief den Test parallel als Referenz zur Validierung. Die Negativkontrolle wurde mit einem Verhältnis Gewicht/Volumen von 1g/5ml Medium extrahiert. Die Positiv-Kontrolle wurde mit einem Verhältnis Gewicht/Volumen von 6$cm^2$/ml des Kulturmediums (DMEM 10% FCS) für 72$\pm$2 Stunden bei 37$\pm$1 °C extrahiert.

Negativ-Kontrolle: Polyethylen (Greiner Cellstart, Art. No. 188271, LOT 04080197).

Positiv-Kontrolle: Puderfreie Industrie-Latex-Handschuhe (Semperit GmbH, LOT 67910077).

Der Test wurde durchgeführt mit L929 Zellen (ATCC No. CCL1, NCTC clone 929 (connective tissue mouse), clone of strain L (DSMZ)). Für den Test wurden Kulturen in 75 $cm^2$ Kulturflaschen (Greiner) in DMEM (PAA) mit 10% FCS (Seromed) bei 37$\pm$1 °C und 5,0% Kohlendioxid verwendet.

Die Zellkulturen werden mit Ca-Mg-freier PBS für ungefähr 3 Minuten behandelt. Die enzymatische Reaktion wird mit DMEM 10% FCS gestoppt und eine einzelne Zell-Suspension mit einer Konzentration von $2 \cdot 10^4$ Zellen/ml hergestellt. 100$\mu$l dieser Suspension werden in die Kavitäten einer Tüpfelplatte gebracht. Die Zellkultur wurde für 24$\pm$2 Stunden bei 37$\pm$1 °C mit 5,0% $CO_2$ und 95% Luft inkubiert.
Anschließend werden Verdünnungen des Extraktes mit DMEM 10% FCS zu den Konzentrationen 100, 80, 50, 30, 20, 10%-Vol in einer weiteren Tüpfelplatte bereitgestellt. Dann wird das Zellkulturmedium der zuvor angesetzten Zellen entfernt und 100$\mu$l der Verdünnungen des Test-Extraktes mit 100$\mu$l der Kontrolle (100% Konzentration) in jeweils drei Proben versetzt. Die Kulturen werden für 24$\pm$2 Stunden bei 37$\pm$1 °C mit 5,0% $CO_2$ und 95% Luft inkubiert.
Die XTT-Einfärbung beginnt 1-2 Stunden vor Ende der Inkubationsperiode. Dazu werden 50$\mu$l der XTT-Farbmixtur (Roche Diagnostics) zu jeder Zellkultur gegeben. Die Mischung besteht aus der XTT-Marker-Reagenz (5 ml) und der Elektronenkopplungsreagenz (0,1 ml). Nach Abschluß der Inkubationszeit (1-2 Stunden) werden die Zellkulturen in einen Platten Detektor (Biotek Systems) zur colorimetrischen Analyse gegeben. Dabei wird die Absorption bei 490 nm registriert und im Vergleich zur Referenzwellenlänge 630 nm ausgewertet.

Eine Reduktion der Anzahl lebender Zellen entspricht einer Abnahme der Aktivität der Dehydrogenase der Mitochondrien in den jeweiligen Zellkulturen. Dadurch wird die Bildung des orangefarbenen Formazan-Farbstoffes in direkter Korrelation reduziert und als Extinktion quantitativ aufgezeichnet.

$$\text{Aktivität der Mitochondrien Dehydrogenase [\%]} = \frac{A(\text{Probe, 490nm}) - A(\text{Referenz, 490nm})}{A(\text{Kontrolle, 490nm}) - A(\text{Referenz, 490nm})}$$

A(Probe, 490nm) Absorption mit Prüfextrakt
A(Referenz, 490nm) Absorption eines leere Mediums (ohne Zellen)
A(Kontrolle, 490nm) Absorption mit Kontrollkultur ohne Extrakt

Das Ergebnis wurde als arithmetisches Mittel mit Standardabweichung aus einem Satz von jeweils drei Proben bestimmt. Eine Dehydrogenaseaktivität unter 70% wird als klar cyctotoxisch gewertet.

### Diskussion der Ergebnisse

Die stärker ausgeprägte Zytotoxizität von Acrylaten gegenüber Methacrylat-Monomeren mit vergleichbarer Molmasse, Polarität und Funtionalisierungsgrad ist bekannt. Aus diesem Grund werden in Dentalmaterialien bevorzugt reine Mischungen verschiedener Methacrylate oder nur geringe Anteile von Acrylatmonomeren eingesetzt.

In Übereinstimmung mit diesem bekannten Fakt zeigen auch die eigenen Untersuchungen mit Anteilen verschiedener Acrylatmonomere (Sartomer 368 und 295) eine nachweisbar höhere Zytotoxizität gegenüber einer vergleichbaren Zubereitung aus Methacrylaten ohne diese Zusätze. Während das pastöse Versuchskomposit 201 einer gebräuchlichen Zusammensetzung eines Dentalharzes aus Bis-GMA und Triethylenglycoldimethacrylat (TEGDMA) im Verhältnis 7:3 entspricht und kein zytotoxisches Potential zeigt, kann bei Probe 204 mit einem Zusatz von multifunktionellen Acrylatmonomeren ein deutlicher Anstieg der Cytotoxizität beobachtet werden.

Entgegen diesem bekannten Effekt zeigte ein vergleichbares Komposit beim Austausch von Bis-GMA gegen das diacrylatfunktionelle TCD-Monomer sogar eine Reduktion der zytotoxischen Wirksamkeit. Das erfindungsgemäße TCD-Monomer reduzierte im klassischen dentalen Kompositwerkstoffen nachweisbar das zytotoxische Potential.

In einer anderen Harzmischung mit Urethanmethacrylat wurden die Versuche reproduziert. Eine Mischung aus Triethylenglycoldimethacrylat, UDMA und dem TCD-Monomer wurde in verschiedenen Kombinationen mit weiteren Monomeren geprüft. Um eine Vergleichbarkeit zum klassischen Bis-GMA / TEGDMA - Komposit zu erreichen wurden in einem Versuch 72% Bis-GMA zugegeben und die Probe 338 geprüft. Mit dem gehärteten Komposit wurde eine sehr

geringes zytotoxisches Potential nachgewiesen, das noch unter der Wirksamkeit von Probe 230 lag. Der vollständige Ersatz des Bis-GMA durch das vergleichbar schrumpfarme Acrylatmonomer TCD-DI-HEA führte in den Proben 349 und 350 zu einem ähnlich günstigen zytotoxischen Potential, wobei der Initiatorgehalt noch reduziert werden konnte aufgrund der höheren Reaktivität des Monomeren.

Damit konnte auch für eine anders zusammengesetzte Harzmischung der gleiche Zusammenhang zwischen zytotoxischer Wirksamkeit und Art der enthaltenen Acrylatmonomere gezeigt werden. In den durchgeführten Versuchen konnte gezeigt werden, dass das erfindungsgemäße Acrylatmonomer mit einer TCD-Urethan-Struktur günstigere toxikologische Eigenschaften bewirkt als die üblicherweise eingesetzten reaktionsträgeren Methacrylatmonomere oder andere reaktive Acrylatmonomere.

Das sehr geringe zytotoxische Potential von härtbaren Dentalwerkstoffen mit dem erfindungsgemäßen Monomer TCD-DI-HEA, die ein Medizinprodukt darstellen und üblicherweise im ständigen Kontakt mit dem lebenden Gewebe verbleiben, ist von zentraler Bedeutung für die Einsetzbarkeit und biologische Akzeptanz solcher Materialien bei Patienten und Anwendern.

**Tabelle I Ergebnisse der Zytotoxizitätsmessungen**

| | | | | | | | | Mitochondriale Hydrogenase Aktivität bei Extraktkonzentration in % | | | | | | |
|---|---|---|---|---|---|---|---|---|---|---|---|---|---|---|
| Type | TCD | Bis GMA | TED-MA | UD-MA | SR 295 Tetra A | UT-MA | SR 368 Tri A | **100** | **80** | **50** | **30** | **20** | **10** | Bewertung |
| **Probe 5** 201 | | 68 % | 32 % | | | | | 88 | 91 | 94 | 97 | 98 | 96 | Kein zytotoxisches Potential |
| **Probe 1** 204 | | 38 % | 13 % | 19 % | 10 % | | 20 % | 0 | 1 | 18 | 71 | 90 | 98 | Ausgeprägtes zytotoxisches Potential |
| **Probe 6** 230 | 80 % | | 20 % | | | | | 86 | 94 | 96 | 99 | 99 | 96 | Kein zytotoxisches Potential |
| **Probe 9** Polymerisat 338 | 13 % | 73 % | 4 % | 6 % | | 4 % | | 94 | 94 | 97 | 98 | 99 | 98 | Kein zytotoxisches Potential |
| **Probe 7** Polymerisat 349 | 90 % | | 2 % | 4 % | | 4 % | | 89 | 92 | 96 | 98 | 100 | 99 | Kein zytotoxisches Potential |
| **Probe 8** Polymerisat 350 | 90 % | | 2 % | 4 % | | 4% | | 91 | 93 | 98 | 100 | 100 | 99 | Kein zytotoxisches Potential |

## Patentansprüche

1. Verwendung eines
Vernetzeranteils, der zu mehr als 50% gebildet wird von Acrylatmonomeren die ausgewählt sind aus Acrylatmonomeren mit einer diacrylatfunktionellen TCD-Urethan-Struktur ausgewählt aus TCD-di-HEA, einem Acrylsäureester des Tricyclo[5.2.1.02.6]decans zur Herstellung einer Dentalkomposit-Zusammensetzung enthaltend Monomere, Vernetzer, Füllstoffe, Initiatoren, wobei
die Cytotoxizität des gehärteten Komposits entsprechend den Normvorgaben nach ISO 10993-5 und DIN EN ISO 7405 die Bewertung "kein zytotoxisches Potential" aufweist.

2. Verwendung nach Anspruch 1, wobei der Vernetzeranteil zusätzlich Silorane enthält.

3. Verwendung nach Anspruch 1, die im Wesentlichen Bis-GMA-frei ist.

## Claims

1. Use of a crosslinking agent which is formed by an amount of more than 50% by acrylate monomers that are selected from acrylate monomers with a diacrylate-functional TCD-urethane monomer structure selected from TCD-di-HEA an acrylic acid ester of tricyclo[5.2,1.02.6]decane for the production of a dental composites comprising monomers, crosslinking agents, fillers, initiators, wherein
the cytotoxicity of the hardened composite corresponding to the standard requirements according to ISO 10993-5 and DIN EN ISO 7405. has the assessment "no cytotoxic potential".

2. Use according to claim 1, the crosslinking agent moiety additionally containing silorans.

3. Use according to claim 1 which is essentially free from bis-GMA,

## Revendications

1. Utilisation d'une proportion d'agent de réticulation qui est formée à plus de 50 % de monomères d'acrylate qui sont sélectionnés parmi des monomères d'acrylate avec une structure TCD-uréthane à fonction diacrylate sélectionnés parmi TCD-di-HEA, un ester d'acide acrylique du tricyclo[5.2.1.02.6]décane pour la fabrication d'une composition de composite dentaire contenant des monomères, agents de réticulation, charges, initiateurs, dans laquelle la cytotoxicité du composite durci correspondant aux exigences de norme selon ISO 10993-5 et DIN EN ISO 7405 présente l'évaluation « aucun potentiel cytotoxique ».

2. Utilisation selon la revendication 1, dans laquelle la proportion d'agent de réticulation contient en outre des siloranes.

3. Utilisation selon la revendication 1, qui est essentiellement exempte de Bis-GMA.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0254185 A
- EP 0934926 A
- DE 10001228
- EP 1117368 A
- EP 1719497 A1